(19) Europäisches Patentamt

European Patent Office

Office européen des brevets

(11) **EP 0 559 164 B1**

(12) **EUROPÄISCHE PATENTSCHRIFT**

(45) Veröffentlichungstag und Bekanntmachung des Hinweises auf die Patenterteilung:
**24.05.2000 Patentblatt 2000/21**

(51) Int. Cl.[7]: **G01N 33/72**
// G01N33/543

(21) Anmeldenummer: **93103349.2**

(22) Anmeldetag: **03.03.1993**

(54) **Immunologische Methode zur Bestimmung von HbAlc**

Immunological method to determine HbAlc

Méthode immunologique pour déterminer HbAlc

(84) Benannte Vertragsstaaten:
**AT BE CH DE DK ES FR GB GR IE IT LI LU NL PT SE**

(30) Priorität: **05.03.1992 DE 4206932**

(43) Veröffentlichungstag der Anmeldung:
**08.09.1993 Patentblatt 1993/36**

(73) Patentinhaber:
**Roche Diagnostics GmbH**
**68298 Mannheim (DE)**

(72) Erfinder:
• **Karl, Johann, Dr.**
  **W-8123 Peissenberg (DE)**
• **Kerscher, Lorenz, Dr.**
  **W-8122 Penzberg (DE)**
• **Schneider, Erich, Dr.**
  **W-6800 Mannheim 31 (DE)**

(56) Entgegenhaltungen:
EP-A- 0 185 870          EP-A- 0 215 401
EP-A- 0 309 883          EP-A- 0 315 864
EP-A- 0 444 241          EP-A- 0 456 088
EP-A- 0 539 923          FR-A- 2 559 267

• **CLINICAL BIOCHEMISTRY, Band 15, Nr. 1, 1982, Toronto, ON (CD); OSHIRO et al., Seiten 83-88/**
• **DATABASE WPIL, Woche 8723, Derwent Publications Ltd., London (GB); AN 87-159653/**

**Beschreibung**

Die Erfindung betrifft eine Methode zur Bestimmung des Gehaltes eines bestimmten Hämoglobinderivats in einer Blutprobe. Speziell betrifft die Erfindung eine Methode zur Bestimmung des Gehaltes von Hämoglobinderivaten, wie glykiertes Hämoglobin, die die separate Bestimmung des Gesamthämoglobingehalts und die Bestimmung des Hämoglobinderivates erfordern, um den Anteil des derivatisierten Hämoglobins im Blut zu bestimmen.

In Abhängigkeit von der Höhe des Blutzuckers verbindet sich ein kleiner Teil der von den Erythrozyten aufgenommenen Glucose in einer nicht enzymatischen Reaktion mit dem N-terminalen Valinrest der $\beta$-Kette des Globins. Die Reaktion zu dem stabilen glykierten Hämoglobinderivat $HbA_{1c}$ (Ketoaminform) verläuft in zwei Stufen. Erstens wird Glucose über eine schnelle reversible Anlagerung an Hämoglobin unter Bildung eines labilen glykierten Hämoglobinderivats (Schiffbase) gebunden. Durch eine irreversible, langsame Umlagerungsreaktion (Amadori-Umlagerung) wird die stabile Form gebildet. Der Anteil von $HbA_{1c}$ am Gesamthämoglobin beträgt bei Stoffwechselgesunden 3 - 6 %. Bei Patienten mit Diabetes kann der Anteil des $HbA_{1c}$ proportional zur Höhe der Blutglucosekonzentration während der vorausgegangenen vier bis zwölf Wochen bis auf Werte von 12 %, vereinzelt sogar bis 20 % ansteigen. Die Bestimmung des relativen Anteils von $HbA_{1c}$ am Gesamthämoglobingehalt bietet sich insbesondere als integraler Parameter zur Verlaufskontrolle der Blutzuckereinstellung an.

Es wurden eine Reihe von Methoden zur Bestimmung von glykiertem Hämoglobin beschrieben. Konventionelle Methoden basieren auf Techniken, wie Elektrophorese, isoelektrische Fokussierung und kolorimetrischen Bestimmungen sowie Ionenaustausch- und Affinitätschromatographie. Nachdem die Herstellung von spezifischen polyklonalen Antikörpern (US-Patent 4,247,533) und monoklonalen Antikörpern (EP-A-0 316 306, EP-A-0 201 187) beschrieben war, wurden eine Reihe von immunologischen Methoden zum Nachweis von $HbA_{1c}$ entwickelt.

Die EP-A-0 185 870 beschreibt eine immunologische Methode zur Bestimmung von Proteinen, u. a. $HbA_{1c}$. Es werden monoklonale Antikörper, die spezifisch lineare Peptidepitope erkennen, benutzt. Zur Epitopfreisetzung wird eine Denaturierung vorgeschrieben, u. a. durch chaotrope Reagenzien. Bei Temperaturen unterhalb 37 °C nimmt dieser Denaturierungsschritt eine bis mehrere Stunden, bei Temperaturen über 50 °C eine Minute in Anspruch. Auf die gleichzeitige Bestimmung des Gesamthämoglobingehaltes in der Probe wird nicht eingegangen.

In der EP-A-0 201 187 wird eine Methode zur Bestimmung von $HbA_{1c}$ beschrieben, bei der monoklonale Antikörper gegen $HbA_{1c}$ eingesetzt werden, die durch Immunisierung mit nativem humanem $HbA_{1c}$ gewonnen wurden. Der Nachweis wird bei Temperaturen von 4 bis 37 °C durchgeführt, wobei jeder Inkubationsschritt bis zu 72 Stunden betragen kann. Eine Bestimmung des Gesamthämoglobin- und des $HbA_{1c}$-Gehaltes in der selben Probe wird nicht beschrieben.

In der EP-A-0 315 864 wird eine Methode zur Bestimmung des relativen Gehaltes an $HbA_{1c}$ in einer Blutprobe beschrieben. Hämoglobin wird durch Thiocyanat denaturiert und durch ein zusätzliches Oxidationsmittel, bevorzugt Ferricyanid in Met-Hämoglobin überführt. In der so behandelten Blutprobe kann der Gesamthämoglobingehalt sowie der Gehalt an $HbA_{1c}$ bestimmt werden.

Der Einsatz von Lithiumsalzen, bevorzugt Lithiumthiocyanat zur Lyse der Erythrozyten und Denaturierung des Hämoglobinderivates wird in der EP-A-0 407 860 beschrieben. Der $HbA_{1c}$ -Gehalt kann immunologisch bestimmt werden. Zum Nachweis des Gesamthämoglobins muß zusätzlich ein Oxidationsmittel, bevorzugt Ferricyanid, das Hämoglobin in Met-Hämoglobin überführt, zugesetzt werden.

Ein Nachteil der beiden letzten Methoden besteht darin, daß zur Überführung von Hämoglobin in Cyano-Met-Hämoglobin Cyanid eingesetzt werden muß. Ein Ersatz des Cyanids durch Natriumlaurylsulfat (SLS) zum Nachweis von Gesamthämoglobin wird in Clin. Biochem. 15 (1982) 83 - 88 beschrieben. Hier wird allerdings kein Hinweis gegeben, daß SLS zur Probenvorbehandlung bei der immunologischen Bestimmung eines Hämoglobinderivates, speziell $HbA_{1c}$, geeignet ist.

Auch in der EP-A-0 184 187 wird ein cyanidfreies Reagenz zur Bestimmung des Gesamthämoglobins in einer Blutprobe beschrieben. Das Reagenz ist ein ionisches oberflächenaktives Mittel mit einem pH von mindestens 11,3, bevorzugt mehr als 13,7. Auf die immunologische Bestimmung eines Hämoglobinderivates wird nicht eingegangen. Der sehr hohe pH-Wert wäre bei der immunologischen Bestimmung eines glykierten Hämoglobinderivates von Nachteil, da im stark alkalischen eine Abspaltung der Zuckeranteile vom Hämoglobin auftreten kann. Im Falle einer Enzymmarkierung der verwendeten Antikörper konnte die Enzymreaktion gehemmt werden, da das pH-Optimum der meistgenutzten Enzyme bei einem pH von 6 bis 8 liegt. Auch die immunologische Reaktion kann durch den hohen pH-Wert gehemmt werden.

JP 58061465, Database WPIL, Week 8723, Derwent Publications Ltd., AN 87-159653 beschreibt eine Methode zur Messung von Glykosyl-Hämoglobin. Ein Hämolysereagenz, ein nichtionisches oder ionisches Detergens, ist auf einer porösen Membran absorbiert.

FR-A 2 559 267 beschreibt eine Methode zum Nachweis von glykiertem Hämoglobin, wobei eine Blutprobe mit einer Lösung von SDS behandelt wird. Danach wird der Glukoserest von $HbA_{1c}$ durch Säurebehandlung abgespalten. Nach der Deproteinisierung wird die Glukose photometrisch bestimmt.

EP-A 0 444 241 beschreibt eine Methode zum Nachwers von Hämoglobin, wobei ein Reagenz eingesetzt wird, das neben anderen Bestandteilen kationische Detergenzien enthält.

Es bestand daher weiterhin ein Bedarf an einer immunologischen Nachweismethode zur Bestimmung eines Hämoglobinderivates in Blut, wobei die Hämolyse bei niedrigen Temperaturen - wie beispielsweise bei Raumtemperatur - durchgeführt werden kann, es keiner langen Inkubationszeit zur Probenvorbereitung bedarf und der Einsatz von umweltschädlichen Reagenzien, wie Cyanid vermieden wird. Außerdem sollte die gleichzeitige Bestimmung von Gesamthämoglobin in dem selben Hämolysat möglich sein, um den Anteil des Hämoglobinderivates am Gesamthämoglobingehalt des Blutes ohne weiteren Hämolyseansatz bestimmen zu können. Aufgabe der vorliegenden Erfindung war es, eine solche immunologische Nachweismethode zur Bestimmung eines Hämoglobinderivates in Blut bereitzustellen.

Die Aufgabe wird durch die in den Ansprüchen näher charakterisierte Erfindung gelöst. Im wesentlichen wird diese Aufgabe gelöst durch eine Methode zur Bestimmung des Gehaltes eines Hämoglobinderivates einer Blutprobe, die dadurch gekennzeichnet ist, daß die Blutprobe mit einem Hämolysereagenz mit einem pH von 5 bis 9.5 behandelt wird, das ein ionisches Detergens enthält, und in der hämolysierten Blutprobe das Hämoglobinderivat immunologisch bestimmt wird.

Ein weiterer Gegenstand der Erfindung ist eine Methode zur Bestimmung des Gehaltes eines Hämoglobinderivates und des Gesamthämoglobins in einer Blutprobe, die darin besteht, daß die Blutprobe mit einem Hämolysereagenz mit einem pH von 5,0 bis 9,5 behandelt wird, das ein ionisches Detergens enthält, in der hämolysierten Probe der Gesamthämoglobingehalt bestimmt wird und in der hämolysierten Probe das Hämoglobinderivat immunologisch bestimmt wird.

Ein weiterer Gegenstand der Erfindung ist ein Hämolysereagenz mit einem pH von 5 bis 9,5 das ein ionisches Detergens enthält, dessen Verwendung zur Probenvorbereitung einer Blutprobe zur immunologischen Bestimmung des Hämoglobinderivates und, falls gewünscht, zur Bestimmung des Gesamthämoglobingehaltes sowie ein Testkit, das das Hämolysereagenz enthält.

Durch die Einwirkung des Hämolysereagenzes werden die in der Blutprobe enthaltenden Erythrozyten lysiert und Hämoglobin in ein spezifisches Hämoglobin-Chromophor überführt. Das entstehende Hämoglobin-Chromophor kann über seine charakteristische Absorption zur Bestimmung des Gesamthämoglobingehaltes und über die immunologische Reaktion des spezifischen Epitops zur Bestimmung des Hämoglobinderivates verwendet werden. Als immunologische Nachweismethode können prinzipiell alle gängigen Methoden angewandt werden. Direkte oder indirekte (kompetitive) Methoden wie z. B. Sandwichtests, IEMA-Tests, Präzipitations- oder Agglutinationstests sowie Tests nach dem FPIA- und CEDIA-Prinzip Sowie Naß- als auch Trockentests sind möglich.

Im Hämolysereagenz können als ionische Detergenzien anionische Detergenzien, bevorzugt Natriumdodecylsulfat (SDS), Natriumdioctylsulfosuccinat (DONS), kationische Detergenzien, bevorzugt Tetradecyltrimethylammoniumbromid (TTAB) oder Cetyltrimethylammoniumbromid (CTAB) oder zwitterionische Detergenzien, bevorzugt Zwittergent 3-14, verwendet werden. Das Hämolysereagenz wird der Blutprobe in einer Menge zugegeben, die ausreicht, die Erythrozyten zu lysieren, Hämoglobin in den spezifischen Hämoglobin-Chromophor zu überführen und die Epitope des Hämoglobinderivates freizusetzen. Das Hämolysereagenz wird der Blutprobe in einem Verhältnis von 1:10 bis 1:400 zugegeben, so daß die Konzentration des ionischen Detergenzes in der resultierenden Mischung 0,01 bis 5 Gew. %, bevorzugt 0,5 - 1,5 Gew. % beträgt. Der pH-Wert des Hämolysereagenzes liegt im leicht sauren bis schwach alkalischen Bereich. Bevorzugt liegt der pH zwischen 5,0 bis 9,5, besonders bevorzugt bei 7,4. Zur Einstellung des pH-Wertes im Hämolysereagenz können alle gängigen Puffer verwendet werden. Bevorzugt werden HEPES, MES , TRIS oder Phosphat-Puffer genutzt.

Das Hämolysereagenz kann weitere Reagenzien enthalten, die z. B. der Entstörung der immunologischen Reaktion, der Oxidation des Hämoglobins, der Trübungsbeseitigung, der Stabilisierung oder Konservierung dienen. Einige Detergenzien, u. a. SDS, können immunologische Reaktionen stören. In seltenen Fällen treten nach der Zugabe des Hämolysereagenzes Trübungen und Ausflockungen aufs die unterschiedliche Ursache haben. Bei niedrigen Temperaturen ist beispielsweise SDS schwer löslich. Es hat sich überraschenderweise gezeigt, daß diese Störungen durch die Zugabe eines nichtionischen Detergenzes, bevorzugt sind Polyoxyethylenether, wie Brij® 35 und 58 oder Triton® X100 sowie Polyoxyethylenester, wie Myrj® 52 und 59 oder Tween® 20, vermieden werden können. Normalerweise wird das nichtionische Detergens dem Hämolysereagenz in einer Menge zugegeben, so daß nach Zugabe zu der Probe in der resultierenden Mischung die Konzentration 0,01 bis 5 Gew. %, bevorzugt 0,1 - 0,5 Gew. %, beträgt.

Das weiterhin gebräuchliche Oxidationsmittel Ferricyanid, beispielsweise Hexacyanoferrat (III), kann in dem Hämolysereagenz enthalten sein. Zusammen mit kationischen Detergenzien können allerdings Ausfällungen auftreten. Zusätzlich ist bei der Anwendung von Ferricyanid ein Lichtschutz erforderlich. Es hat sich überraschenderweise gezeigt, daß bei einem Zusatz von Konservierungsmitteln, deren Wirkmechanismus auf einem oxidativen Angriff auf Thiolgruppen beruht, wie beispielsweise Methylisothiazolon oder Bronidox ® K, auf die Zugabe von Ferricyanid gänzlich verzichtet werden kann. Zusammen mit kationischen Detergenzien führen diese Konservierungsmittel zu einem braungrün gefärbten Hämoglobin-Chromophor, dessen Absorptionsmaximum bei 570 nm liegt. Der besondere Vorteil liegt darin, daß erstens durch den Zusatz dieser Konservierungsmittel sowohl die Überführung des Hämoglobins in ein spezifisches Hämoglobin-Chromophor, als auch eine gute Konservierung des Hämolysereagenzes erreicht wird und zweitens der Endpunkt der Hämolyse durch einen Farbumschlag von rot nach braun-grün gut erkennbar ist. Die Konservierungsmittel werden im Hämolysereagenz in einer Konzentration von 0,005 - 0,2 Gew. %, bevorzugt 0,01 - 0,02 Gew. % eingesetzt.

Die Hämolyse, die Überführung von Hämoglobin in das spezifische Hämoglobin-Chromophor sowie die Probenvorbereitung zum Nachweis der Hämoglobinderivate durch das Hämolysereagenz sind bei niedrigen Temperaturen, bevorzugt bei 4 bis 37 °C und besonders bevorzugt bei Raumtemperatur (20 °C) nach 1 bis 10 Minuten abgeschlossen. In den meisten Fällen genügt eine Inkubation von 2 Minuten zur vollständigen Probenvorbereitung.

Die so vorbereitete Blutprobe wird anschließend mit einem Reaktionspuffer im Verhältnis 1:10 bis 1:100 verdünnt. Als Puffer können alle gängigen Puffer verwendet werden, die die immunologische Reaktion nicht stören. Vorzugsweise werden MES- oder HEPES-Puffer in Konzentrationen von 10 bis 200 mmol/1 eingesetzt. Der pH-Wert des Reaktionspuffers beträgt 5,0 bis 8,0. Falls der immunologische Nachweis eine Enzymreaktion beinhaltet, wird der Reaktionspuffer vorzugsweise einen pH-Wert entsprechend dem pH-Optimum der Enzymreaktion besitzen. Der Reaktionspuffer kann weiterhin bereits die für den Hämoglobinderivatnachweis erforderlichen Reagenzien, vor allem die spezifischen Bindepartner, bevorzugt hochspezifische poly- oder monoklonale Antikörper, enthalten.

Es hat sich auch hier überraschenderweise gezeigt, daß die Zugabe eines weiteren Detergenzes, bevorzugt nichtionische Detergenzien wie Brij[®] oder Myrj[®], vorteilhaft ist, um optimale Bedingungen sowohl für die Probenvorbereitung zum Nachweis des Hämoglobinderivates, wie glykiertes Hämoglobin, als auch für die immunologische Reaktion zu bieten und Störungen zu vermeiden. Das Detergens wird dem Reaktionspuffer in einer Menge zugegeben, daß in der resultierenden Mischung die Konzentration 0,01 bis 5 Gew. %, bevorzugt 0,5 Gew. % beträgt.

Falls in der selben Probe neben der Hämoglobinderivatbestimmung der Gesamthämoglobingehalt ermittelt werden soll, wird bevorzugt nach der Zugabe des Reaktionspuffers zur Ermittlung des Gesamthämoglobingehaltes die Absorption bei Wellenlängen von 400 bis 650 nm, bevorzugt 500 bis 650 und besonders bevorzugt bei 546 oder 570 nm gemessen. Im Falle eines Naßtestes wird die Extinktion bei dieser Wellenlänge in der Küvette photometrisch bestimmt, im Falle eines Trockentestes kann die Absorption reflektionsphotometrisch nach Auftragen der resultierenden Mischung auf einen Testträger bestimmt werden.

Ein solcher Testträger zur Bestimmung des Gesamthämoglobingehaltes kann sehr einfach konstruiert werden, da er keine Chemikalien enthalten muß. Ein saugfähiges Vlies, das auf einer transparenten Trägerfolie aufgebracht ist, reicht für einfache Anforderungen aus. Zum Zwecke einer besseren Handhabung und Auswertung des Teststreifens können noch weitere Trägerschichten enthalten sein, z. B. ein Transportvlies oder ein Dosiervlies.

Der Gesamthämoglobingehalt und der Hämoglobinderivatgehalt können auch in unterschiedlichen Portionen der Probe nach Zugabe des Hämolysereagenzes bestimmt werden. In diesem Fall wird in einem Teil des Gemisches aus Probe und Hämolysereagenz, falls erforderlich nach entsprechender Verdünnung, der Gesamthämoglobingehalt photometrisch bestimmt. Zu dem zweiten Teil des Gemisches wird der Reaktionspuffer zugesetzt und anschließend das Hämoglobinderivat immunologisch bestimmt.

Zur Bestimmung des Hämoglobinderivates, wie HbA$_{1c}$, sind prinzipiell alle gängigen immunologischen Methoden geeignet. Wie zuvor beschrieben, können poly- und monoklonale Antikörper gegen Hämoglobinderivate, bevorzugt HbA$_{1c}$ hergestellt werden, die die charakteristischen Epitope des Hämoglobinderivates spezifisch binden (US 4,247,533, EP-A-0 316 306 und EP-A-0 201 187).

Bei der Variante des immunologischen Nachweises, der Art der Markierung sowie der Methode der Detektion der Meßsignale handelt es sich um bekannte Methoden des Standes der Technik. Beispielsweise sind direkte oder indirekte (kompetitive) Methoden wie Sandwichtests mit Enzymmarkierungen (ELISA), IEMA-Testführungen, RIAs-, Präzipitations- und Agglutinationstests sowie homogene Immunoassays wie die CEDIA[®] -, EMIT- oder FPIA-Technologie geeignet.

Im Naßtest erfolgt die immunologische Bestimmung des Hämoglobinderivates bevorzugt nach dem TINIA-Prinzip, da hier kein Separationsschritt notwendig ist. Die Probe wird mit einem analytspezifischen Antikörper und einem Polyhapten als Agglutinationsmittel versetzt. Das Polyhapten besteht aus einem Trägermaterial, z. B. Albumin, Dextran oder IgG, an das mehrere Epitope, an die die spezifischen Antikörper binden können, gekoppelt sind. Als Epitope werden die Hämoglobinderivatspezifischen Strukturen, d.h. glykosylierte Peptide, bevorzugt verwendet. Da die Präzipitation der Antikörper nur über das Polyhapten erfolgen kann, erhält man mit abnehmendem Gehalt an Analyt in der Probe eine zunehmende Trübung, die nephelometrisch oder turbidimetrisch nachgewiesen werden kann.

Vorzugsweise ist der Hämoglobinderivat-spezifische Antikörper bereits im Reaktionspuffer enthalten. Nach der Zugabe des Reaktionspuffers kann in der resultierenden Mischung zunächst der Gesamthämoglobingehalt photometrisch bestimmt werden. Die Präzipitationsreaktion wird durch Zugabe einer Lösung, die das Polyhapten enthält, gestartet. Nach einer kurzen Inkubationszeit, wobei 5 Minuten meist ausreichend sind, kann die resultierende Trübung bei geeigneter Wellenlänge, bevorzugt bei Wellenlängen von 340 bis 600 nm, besonders bevorzugt bei 340 nm, gemessen und hieraus die Hämoglobinderivatkonzentration bestimmt werden. Da die Messung von Gesamthämoglobin bei Wellenlängen von 500 bis 650 nm, bevorzugt bei 546 oder 570 nm, erfolgt, stören sich beide Messungen nicht und können in einer Küvette in der selben Reaktionslösung hintereinander durchgeführt werden.

Bei der Herstellung der Polyhaptenlösung, das heißt der flüssigen galenischen Form des Polyhaptens, das heißt einem Trägermaterial, an das glykosylierte Peptide gebunden sind, hat sich gezeigt, daß dieses in dem üblicherweise verwendeten Phosphatpuffer bei einem pH von 7,0 - 7,5 nicht stabil ist. Solche Instabilitäten von glykosylierten Proteinen im Phosphatpuffer sind beispielsweise aus Ahmed et al., J. Biol. Chem. 261 (1986), 4889 - 4894 bekannt. Durch die Verwendung eines MES-Puffers in einer Konzentration von 5 bis 200 mmol/l, bevorzugt 20 - 50 mmol/l und einem pH-Wert von 5,0 - 8,0, bevorzugt zwischen 6,0 bis 6,5 und einer gleichzeitigen Zugabe von EDTA oder einem äquivalenten Komplexbildner in einer bevorzugten Konzentration von 1 - 50 mmol/l, besonders bevorzugt 0,1 - 20 mmol/l, wurde eine Stabilisierung der glykosylierten Peptide, Proteine und Polyhaptene erzielt. Ein Zusatz von Rinderserumalbumin (RSA) in einer bevorzugten Konzentration von 0,1 - 2 %, besonders bevorzugt 1 %, übte einen weiteren positiven Effekt auf die Stabilität der Polyhaptenlösung aus. Diese Polyhaptenlösung kann mehr als 12 Monate bei 4 °C gelagert werden, ohne daß nennenswerte Instabilitäten des Polyhaptens beziehungsweise der glykosylierten Proteine oder Peptide zu beobachten waren. Bei einer Stressbelastung von 3 Wochen 35 °C treten lediglich Signalabfälle von 15 - 20 % des ursprünglichen Extinktionssignals auf. Es hat sich gezeigt, daß diese Methode zur Lösungsstabilisierung auch bei Lösungen von Glykoproteinen oder glykosylierten Peptiden erfolgreich angewendet werden kann.

Als Trockentest erfolgt die immunologische Bestimmung des Hämoglobinderivates bevorzugt nach dem IEMA-Testprinzip. Nach Zugabe des Reaktionspuffers zu der hämolysierten Blutprobe wird der spezifische enzymmarkierte Antikörper zugesetzt. In einer bevorzugten Ausführungsform ist in dem Reaktionspuffer bereits der Antikörper enthalten. Nach einer kurzen Inkubation wird das Gemisch auf einen Testträger aufgebracht. An einer Epitopmatrix, d. h. einer Testträgerzone, an die mehrere Epitope, an die die spezifischen Antikörper binden können, gekoppelt sind, werden die freien enzymmarkierten Antikörper abgefangen. In einer weiteren Zone, der Nachweiszone, wird der Komplex aus dem Hämoglobinderivat und dem markierten Antikörper durch Umsetzung eines geeigneten Enzymsubstrates reflexionsphotometrisch bestimmt. Das Substrat kann in der Nachweiszone enthalten sein oder durch Inkontaktbringen der Nachweiszone mit einer weiteren Zone, die das Substrat enthält, in diese gelangen.

Zweckmäßig werden die zur erfindungsgemäßen Methode notwendigen Reagenzien in Form eines Testkits in den Handel gebracht, das in mindestens zwei voneinander getrennten Verpackungen neben dem Hämolysereagenz die anderen Reagenzien in gelöster oder lyophylisierter Form oder auf einem Testträger aufgebracht enthält.

Die vorliegende Erfindung wird durch folgende Beispiele verdeutlicht:

Beispiel 1:

Gleichzeitige Bestimmung von Gesamthämoglobin und HbA$_{1c}$ im Naßtest

Die Versuche wurden an einem Hitachi 717 der Firma Boehringer Mannheim GmbH durchgeführt. Der Gesamthämoglobingehalt wurde photometrisch bei einer Wellenlänge von 546 nm gemessen. Der immunologische Nachweis von HbA$_{1c}$ erfolgte nach dem TINIA-Testprinzip durch turbidimetrische Messung bei 340 nm. Alle Bestimmungen und Inkubationen wurden bei einer Temperatur von 37 °C durchgeführt.

Folgende Lösungen wurden verwendet:

Hämolysereagenz:

| | |
|---|---|
| 20 mM | Natriumphosphat-Puffer pH 7,0 |
| 1,0 % | SDS |
| 0,1 % | Natriumazid |
| 0,02 % | Kaliumhexacyanoferrat (III) |
| 0,5 % | Brij® 35 |

Reaktionspuffer:

| 20 mM | MES-Puffer pH 6,0 |
|---|---|
| 150 mM | Natriumchlorid |
| 3,0 % | PEG 6000 |
| 0,5 % | Brij® 35 |
| 0,1 % | Rinderserum Albumin |
| 0,1 % | Natriumazid |
| 10 mM | EDTA |
| 6 mg/ml | PAK ⟨HbA$_{1c}$⟩ S-IgG (DE) (polyklonale Schaf-AK gegen HbA$_{1c}$) oder |
| 100 µg/ml | MAK ⟨HbA$_{1c}$⟩ M-IgG (DE) (monoklonale Maus-AK gegen HbA$_{1c}$) |

Polyhaptenlösung:

| 20 mM | MES-Puffer pH 6,0 |
|---|---|
| 150 mM | Natriumchlorid |
| 6,0 % | PEG 6000 |
| 0,5 % | Brij® 35 |
| 0,1 % | Rinderserum Albumin |
| 25 µg/ml | Polyhapten HbA$_{1c}$-β-1-4(cys, MHS)-RSA 18:1 |

Zur Blutprobe wurde das Hämolysereagenz in einem Verhältnis von 1:100 hinzugefügt und bei 25 °C 2 Minuten inkubiert. Zu 5 µl hämolysierter Probe wurden 250 µl Reaktionspuffer hinzugefügt. Nach 4 Minuten wurde die Absorption des Gesamthämoglobins bei 546 nm gemessen (E1). Eine Minute später wurde die Absorption bei 340 nm gemessen (E2) und anschließend 50 µl Polyhaptenlösung hinzupipettiert und fünf Minuten inkubiert. Danach wird die Trübung bei 340 nm gemessen (E3).

Zur Bestimmung des HbA$_{1c}$-Wertes in g/dl wird die Extinktionsdifferenz $\Delta E = E3 - k \cdot E2$ gegen die HbA$_{1c}$-Konzentration aufgetragen und grafisch ermittelt.

$$k = \text{Volumenkorrekturfaktor} = \frac{\text{Volumen}_{Probe} + \text{Volumen}_{Reaktionspuffer}}{\text{Gesamtvolumen}}$$

Die Konzentration des Gesamthämoglobins wird aus einem konstanten Faktor K durch Multiplikation mit E1 berechnet (K = molarer Extinktionskoeffizient des Hämoglobin-Chromophors x Verdünnungsfaktor). Als Kalibrator diente EDTA-Vollblut mit bekanntem Hämoglobin- und HbA$_{1c}$-Gehalt. Das EDTA-Vollblut wurde zur Erstellung einer Eichkurve verschieden stark mit Hämolysereagenz verdünnt.

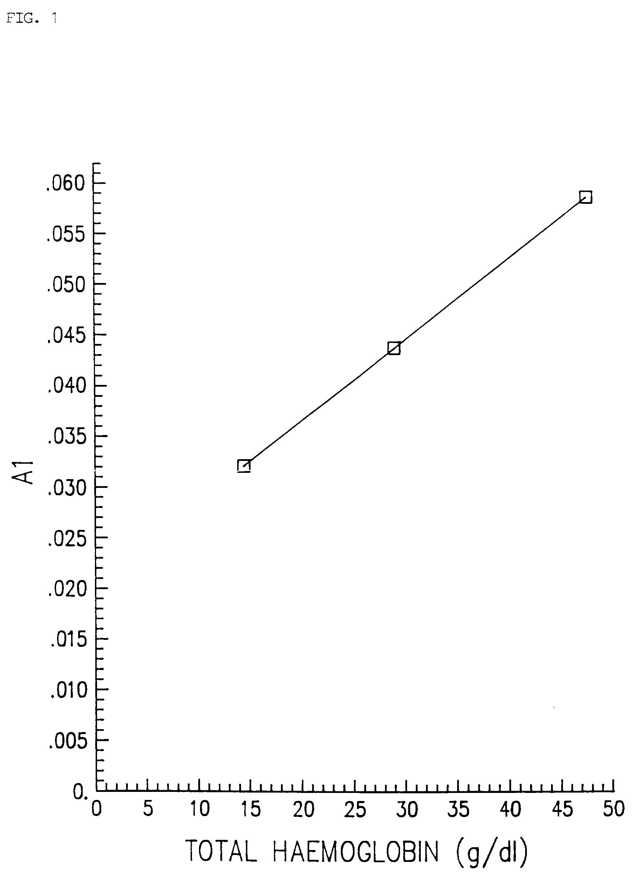

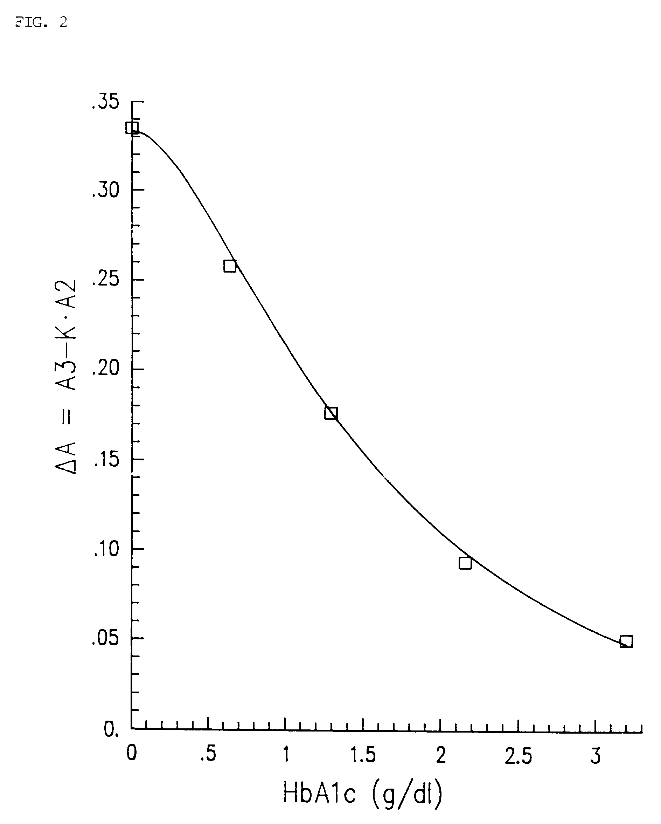

Die Eichkurven sind in Fig. 1 und 2 grafisch dargestellt. In Fig. 1 wurde die Gesamthämoglobinkonzentration gegen E1 aufgetragen. Es ergibt sich eine lineare Beziehung. Bei der Berechnung kann somit mit einem konstanten Faktor K multipliziert werden. In Fig. 2 wurde die Extinktionsdifferenz $\Delta$ E = E3-k $\cdot$ E2 gegen den $HbA_{1c}$-Gehalt aufgetragen.

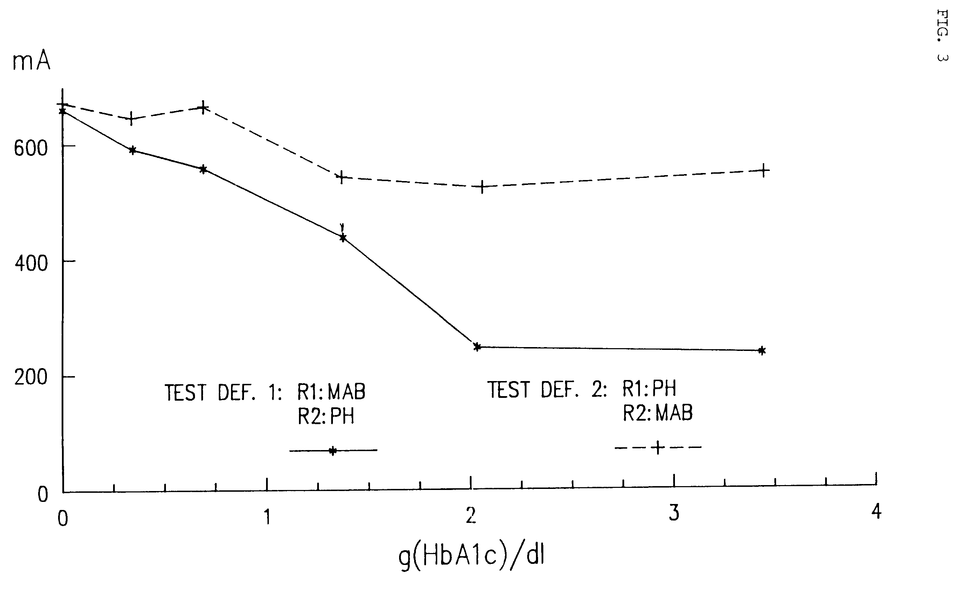

Bei weiteren Versuchen wurde zu der hämolysierten Blutprobe zunächst die Polyhaptenlösung hinzupipettiert und nach einer fünfminütigen Inkubation die Präzipitationsreaktion durch Zugabe des Reaktionspuffers, der den spezifischen Antikörper enthielt, gestartet. Diese Pipettierreihenfolge führte im Vergleich zur oben angegebenen Pipettierreihenfolge, bei der zunächst der Antikörper und danach das Polyhapten zugegeben wurden, zu einer deutlich geringeren Empfindlichkeit bei der $HbA_{1c}$-Bestimmung (Fig. 3).

Beispiel 2

Teststreifen zur Bestimmung von Gesamthämoglobin und $HbA_{1c}$

30 μl Vollblut wurden mit 300 μl Hämolysereagenz versetzt und 10 Minuten bei 20 °C inkubiert. Das Hämolysereagenz bestand aus einer 0,18 %igen SDS-Lösung , die auf pH 7 gepuffert wird.

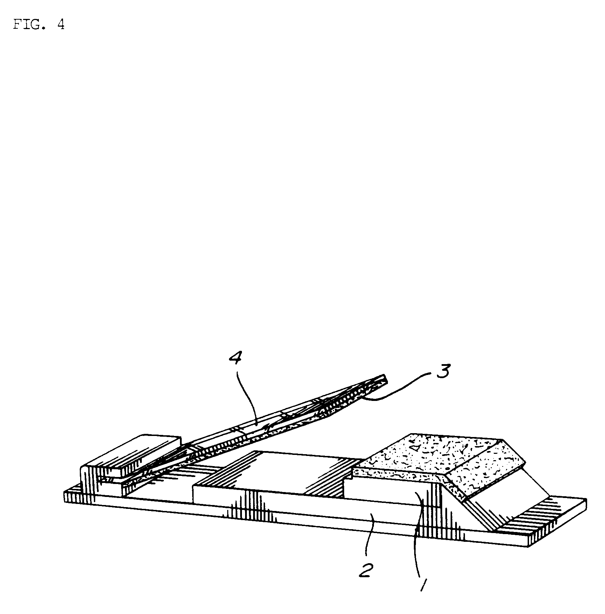

Zur Bestimmung des Gesamthämoglobins wurden 32 μl der hämolysierten Blutprobe auf einen Teststreifen aufgebracht. Der Aufbau des Teststreifens ist in Fig. 4 grafisch dargestellt. Er besteht lediglich aus einem unbehandelten Dosiervlies (1) aus Glasfasern, einem unbehandelten Glasfasertransportvlies (2), einem unbehandelten Polyestergewebe (3) und einer transparenten Polycarbonatfolie (4). Der Teststreifen enthält keine zusätzlichen Chemikalien. Mit dieser Versuchsdurchführung läßt sich der Gesamthämoglobingehalt der Blutprobe bei einer Wellenlänge von 576 nm sehr präzise messen.

Es ergaben sich Variationskoeffizienten unter 2,5 % (Tabelle 1).

Tabelle 1

| Gesamthämoglobin-Bestimmung | |
|---|---|
| Der VK wurde jeweils aus 10 Einzelbestimmungen errechnet. | |
| Hb-Konzentration [g/dl] | VK [%] |
| 14,1 | 1,0 |
| 17,5 | 2,5 |
| 13,7 | 1,9 |
| 14,7 | 1,8 |

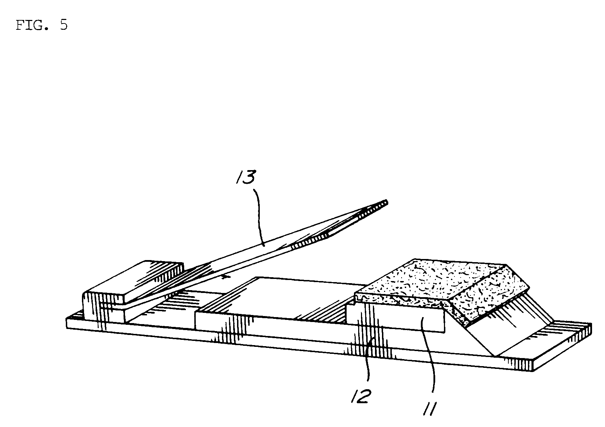

Zur Bestimmung der $HbA_{1c}$-Konzentration werden zu 32 μl der hämolysierten Probe 1200 μl Reaktionspuffer zugesetzt und 10 Minuten inkubiert. Der Reaktionspuffer besteht aus 100 mM Hepes, pH 7,4, 100 mM NaCl und 0,1 % Brij[®] 35 mit dem MAK-Enzymkonjugat. 32 μl des Gemisches werden auf den Teststreifen, der in Fig. 5 grafisch dargestellt ist, aufgetragen. An einer Epitopmatrix (11) wird freies Antikörperenzymkonjugat abgefangen. In der daran anschließenden Zone, dem Transportvlies (12), wird der $HbA_{1c}$-Antikorper-Enzymkomplex durch Umsetzung des Substrates nach Inkontaktbringen des Substratvlieses (13), das das Substrat enthält, mit dem Transportvlies (12) reflexionsphotometrisch bestimmt (Tabelle 2).

Tabelle 2

| Bestimmung der $HbA_{1c}$-Konzentration mittels eines Teststreifens | |
|---|---|
| Das VK wurde aus jeweils 10 Einzelmessungen ermittelt. | |
| $HbA_{1c}$-Konzentration [g/dl] | VK [%] |
| 0,8 | 6,8 |

Tabelle 2 (fortgesetzt)

| Bestimmung der HbA$_{1c}$-Konzentration mittels eines Teststreifens | |
|---|---|
| Das VK wurde aus jeweils 10 Einzel-messungen ermittelt. | |
| 1,1 | 5,4 |
| 1,4 | 6,0 |

Beispiel 3:

Einfluß verschiedener ionischer Detergenzien auf die Bestimmung von HbA$_{1c}$

Die Versuche wurden wie im Beispiel 1 beschrieben durchgeführt. Das Hämolysereagenz besaß folgende Zusammensetzung:

| 20 mM | Natriumphosphat-Puffer pH 7,2 |
|---|---|
| 0,1 % | Natriumazid |
| 0,02 % | Kaliumhexacyanoferrat (III) |
| 0,5 % | Brij[®] 35 |

Die in der Tabelle 3 angegebenen ionischen Detergenzien waren jeweils in der dort angegebenen Konzentration enthalten.

Im Falle von TTAB und CTAB war im Hämolysereagenz kein Kaliumhexacyanoferrat (III) enthalten, da dies mit diezen Detergenzien ausfällt.

Die Zusammensetzung des Reaktionspuffers und der Polyhaptenlösung entspricht dem Beispiel 1.

Als Blutprobe wurde ein Standard mit einem HbA$_{1c}$-Gehalt von 3,2 g/dl verwendet. Alle getesteten ionischen Detergenzien bewirkten eine Lyse der Zellen und eine Epitopfreisetzung. Am geeignetsten erwies sich das anionische Detergens SDS sowie die kationischen Detergenzien TTAB und CTAB.

Tabelle 3

| HbA$_{1c}$-Bestimmung Einfluß verschiedener ionischer Detergenzien im Hämolysereagenz. | | | | |
|---|---|---|---|---|
| Die Absorption wurde bei 340 nm gemessen. | | | | |
| Detergens | Konzentration im Hämolysereagenz | E1 [0 g/dl HbA$_{1c}$] | E2 [3,2 g/dl HbA$_{1c}$] | $\Delta E = E1-E2$ |
| SDS | 0,5 % | 337 mE | 73 mE | 264 mE |
| SDS | 1,0 % | 338 mE | 80 mE | 258 mE |
| Zwittergent[®] 3-14 | 0,5 % | 341 mE | 172 mE | 169 mE |
| Zwittergent[®] 3-14 | 1,0 % | 343 mE | 139 mE | 204 mE |
| TTAB | 1,0 % | 377 mE | 149 mE | 228 mE |
| CTAB | 1,0 % | 374 mE | 137 mE | 237 mE |
| Kontrolle o. ionisches Detergens | -- | 340 mE | 275 mE | 65 mE |

Beispiel 4:

Abhängigkeit des dynamischen Meßbereiches der HbA$_{1c}$-Bestimmung von der Brij[®] 35-Konzentration

Die Versuche wurden wie im Beispiel 3 durchgeführt. Im Hämolysereagenz wurde konstant 1 % SDS eingesetzt. Die Konzentration von Brij[®] 35 im Reaktionspuffer wurde innerhalb dem in Tabelle 4 genannten Bereich variiert. Als Probe diente der im Beispiel 3 genannte HbA$_{1c}$-Standard.
Der Meßbereich erweiterte sich von 47 mE der Kontrolle schon durch die Zugabe von 0,1 % Brij[®] 35 auf 233 mE. Die bevorzugte Konzentration von Brij[®] 35 lag bei 0,1 bis 1,0 %.

Tabelle 4

| Einfluß verschiedener Brij[®]-Konzentrationen im Reaktionspuffer auf die HbA$_{1c}$-Bestimmung | | | |
|---|---|---|---|
| Die Absorption wurde bei 340 nm gemessen | | | |
| Konzentration Brij[®] 35 im Reaktionspuffer | E1 [0 g/dl HbA$_{1c}$] | E2 [3,2 g/dl HbA$_{1c}$] | ΔE=E1-E2 |
| - | 240 mE | 193 mE | 47 mE |
| 0,1 % | 266 mE | 33 mE | 233 mE |
| 0,25 % | 274 mE | 40 mE | 234 mE |
| 0,5 % | 302 mE | 57 mE | 245 mE |
| 1,0 % | 351 mE | 99 mE | 252 mE |
| 2,0 % | 385 mE | 242 mE | 143 mE |

Beispiel 5:

Einfluß verschiedener Detergenzien im Reaktionspuffer auf die HbA$_{1c}$-Bestimmung

Die Versuche wurden Analog Beispiel 4 durchgeführt. Im Reaktionspuffer wurden die in der Tabelle 5 angeführten Detergenzien eingesetzt. Durch die Zugabe von Brij[®] 35, 56 und 58 sowie Myrj[®] 52 und 59 wurde der dynamische Meßbereich am weitesten ausgedehnt. Aber auch andere nichtionische Detergenzien, wie Triton[®], sowie zwitterionische Detergenzien, wie Zwittergent[®] 3=14 zeigten im Vergleich zur Kontrolle einen deutlich ausgeprägten positiven Effekt.

Tabelle 5

| Einfluß von Detergenzien im Reaktionspuffer | | | | |
|---|---|---|---|---|
| Detergens | Konzenträtion im Reaktionspuffer | E1 [0 g/dl HbA$_{1c}$] | E2 [3,2 g/dl HbA$_{1c}$] | Δ E = E1-E2 |
| Kontrolle | - | 240 mE | 193 mE | 47 mE |
| Brij[®] 35 | 0,5 % | 302 mE | 57 mE | 245 mE |
| Brij[®] 56 | 0,5 % | 302 mE | 57 mE | 245 mE |
| Brij[®] 58 | 0,5 % | 303 mE | 51 mE | 252 mE |
| Triton[®] x 114 | 0,2 % | 523 mE | 275 mE | 248 mE |
| Triton[®] x 100 | 0,5 % | 298 mE | 202 mE | 96 mE |
| Tween[®] 80 | 0,25 % | 277 mE | 143 mE | 134 mE |
| Tween[®] 60 | 0,25 % | 266 mE | 75 mE | 191 mE |
| Tween[®] 40 | 0,25 % | 276 mE | 86 mE | 189 mE |

Tabelle 5 (fortgesetzt)

| Einfluß von Detergenzien im Reaktionspuffer | | | | |
|---|---|---|---|---|
| Detergens | Konzentration im Reaktionspuffer | E1 [0 g/dl $HbA_{1c}$] | E2 [3,2 g/dl $HbA_{1c}$] | $\Delta E = E1-E2$ |
| Tween[®] 20 | 0,1 % | 266 mE | 131 mE | 135 mE |
| Zwittergent[®] 3-14 | 0,1 % | 308 mE | 155 mE | 153 mE |
| Myrj[®] 52 | 0,5 % | 308 mE | 40 mE | 268 mE |
| Myrj[®] 59 | 0,5 % | 317 mE | 31 mE | 286 mE |

Beispiel 6:

Gleichzeitige Bestimmung von Gesamthämoglobin und $HbA_{1c}$ mit dem spezifischen braun-grünen Hämoglobin-Chromophor

Die Versuche wurden an einem Hitachi 717 der Firma Boehringer Mannheim GmbH durchgeführt. Der Gesamthämoglobingehalt wurde photometrisch bei einer Wellenlänge von 570 nm gemessen. Der immunologische Nachweis von $HbA_{1c}$ erfolgte nach dem TINIA-Testprinzip durch turbidimetrische Messung bei 340 nm. Alle Bestimmungen und Inkubationen wurden bei einer Temperatur von 37 °C durchgeführt.
Folgende Lösungen wurden verwendet:

Hämolysereagenz:

| | |
|---|---|
| 20 mM | Natriumphosphat-Puffer pH 7,4 |
| 1,0 % | Tetradecyltrimethylammoniumbromid (TTAB) |
| 0,01 % | Methylisothiazolon |
| 0,02 % | Bronidox [®] K |
| 0,5 % | Brij[®] 35 |
| 10 mM | EDTA |

Reaktionspuffer:

| | |
|---|---|
| 20 mM | MES-Puffer pH 6,0 |
| 150 mM | Natriumchlorid |
| 3,0 % | PEG 6000 |
| 0,5 % | Brij[®] 35 |
| 0,01 % | Methylisothiazolon |
| 0,02 % | Bronidox [®] K |
| 1,2 mg/ml | PAK 〈$HbA_{1c}$〉 S-IgG (DE) (polyklonale Schaf-AK gegen $HbA_{1c}$) |

Polyhaptenlösung:

| 20 mM | MES-Puffer pH 6,0 |
|---|---|
| 150 mM | Natriumchlorid |
| 6,0 % | PEG 6000 |
| 0,5 % | Brij® 35 |
| 20 μg/ml | Polyhapten |

Zur Blutprobe wurde das Hämolysereagenz in einem Verhältnis von 1:100 hinzugefügt und bei 25 °C 2 Minuten inkubiert. Zu 10 μl hämolysierter Probe wurden 250 μl Reaktionspuffer hinzugefügt. Nach 4 Minuten wurde die Absorption des Gesamthämoglobins bei 570 nm gemessen (E1). Eine Minute später wurde die Absorption bei 340 nm gemessen (E2) und anschließend 50 μl Polyhaptenlösung hinzupipettiert und fünf Minuten inkubiert. Danach wird die Trübung bei 340 nm gemessen (E3).

Zur Bestimmung des $HbA_{1c}$-Wertes in g/dl wird die Extinktionsdifferenz $\Delta E = E3 - k \cdot E2$ gegen die $HbA_{1c}$-Konzentration aufgetragen und grafisch ermittelt.

$$k = Volumenkorrekturfaktor = \frac{Volumen_{Probe} + Volumen_{Reaktionspuffer}}{Gesamtvolumen}$$

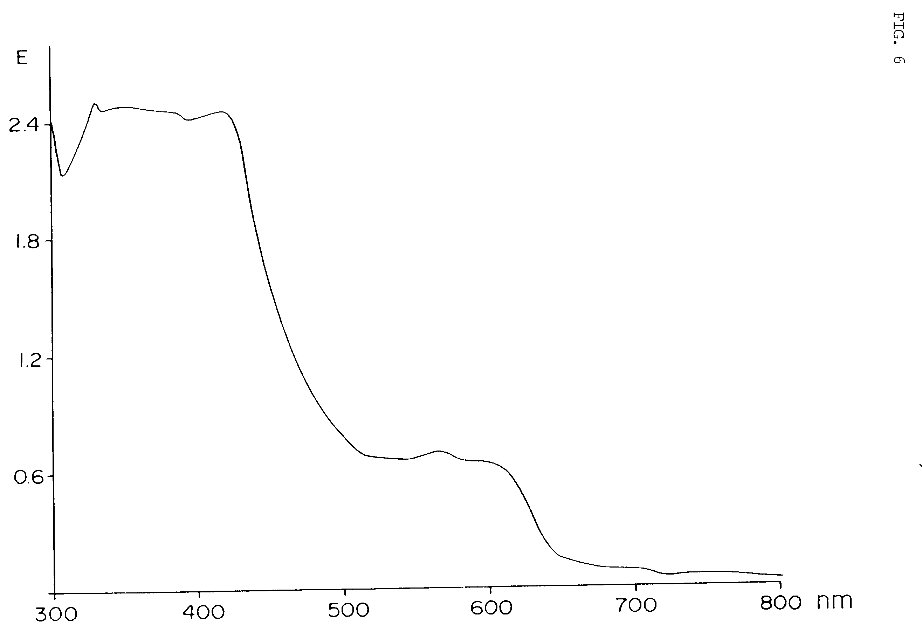

Die Konzentration des Gesamthämoglobins wird aus einem konstanten Faktor K durch Multiplikation mit E1 berechnet (K = molarer Extinktionskoeffizient des Hämoglobin-Chromophors x Verdünnungsfaktor). Das charakteristische Absorptionsspektrum ist in Fig. 6 wiedergegeben. Als Kalibrator diente EDTA-Vollblut mit bekanntem Hämoglobin- und $HbA_{1c}$-Gehalt. Das EDTA-Vollblut wurde zur Erstellung einer Eichkurve verschieden stark mit Hämolysereagenz verdünnt.

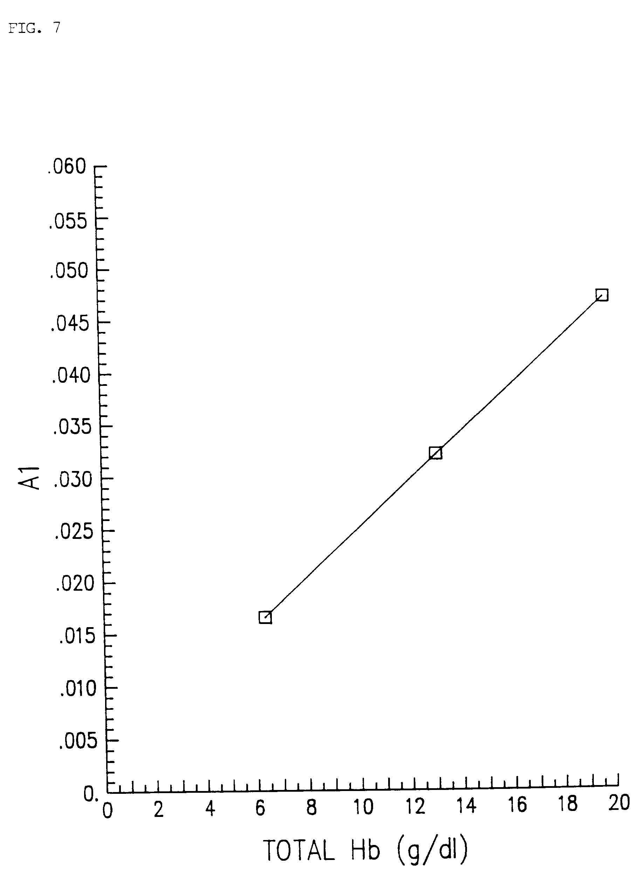

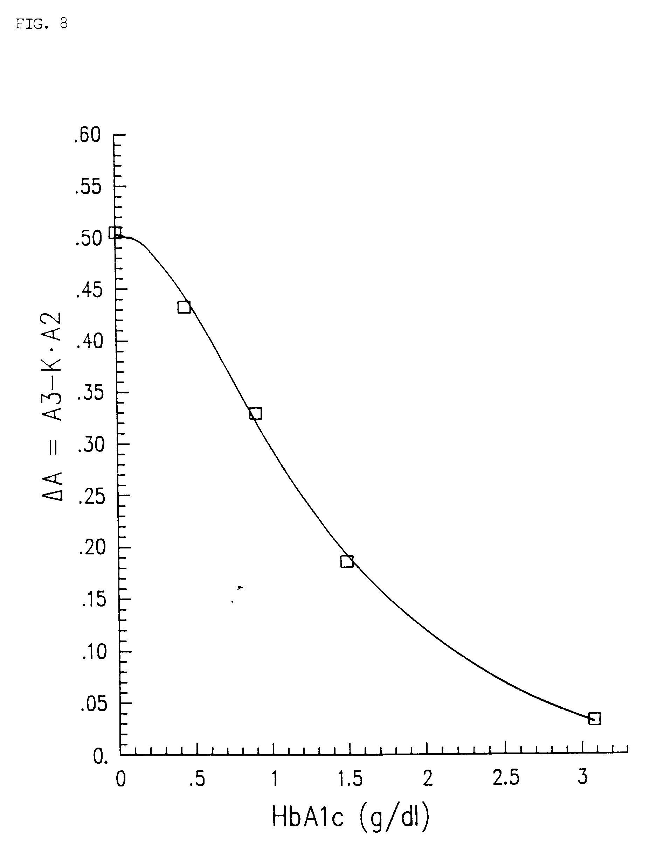

Die Eichkurven sind in Fig. 7 und 8 grafisch dargestellt. In Fig. 7 wurde die Gesamthämoglobinkonzentration gegen E1 aufgetragen. Es ergibt sich eine lineare Beziehung. Bei der Berechnung kann somit mit einem konstanten Faktor K multipliziert werden. In Fig. 8 wurde die Extinktionsdifferenz $\Delta E = E3 - k \cdot E2$ gegen den $HbA_{1c}$-Gehalt aufgetragen.

**Patentansprüche**

**1.** Methode zur Bestimmung des Gehaltes an glykiertem Hämoglobin in einer Blutprobe, dadurch gekennzeichnet, daß

    a) die Blutprobe mit einem Hämolysereagenz mit einen pH von 5 bis 9,5 bei Temperaturen von 4 bis 37° C bis zu 10 Minuten behandelt wird, das ein ionisches Detergens enthält. und

    b) in der hämolysierten Blutprobe das glykierte Hämoglobin immunologisch bestimmt wird.

**2.** Methode nach Anspruch 1, dadurch gekennzeichnet, daß das Hämolysereagenz zusätzlich ein nichtionisches Detergens enthält.

**3.** Methode gemäß einem der Ansprüche 1 - 2, dadurch gekennzeichnet, daß die hämolysierte Probe mit einem Reaktionspuffer versetzt wird, der ein nichtionisches oder zwitterionisches Detergens enthält und in der resultierenden Mischung das glykierte Hämoglobin immunologisch bestimmt wird.

4. Methode gemäß einem der Ansprüche 1 - 3, dadurch gekennzeichnet, daß das glykierte Hämoglobin HbA$_{1c}$ ist.

5. Methode zur Bestimmung des Gehaltes eines glykierten Hämoglobins und des Gesamthämoglobins aus einer identischen Blutprobe, dadurch gekennzeichnet, daß

   a) die Blutprobe mit einem Hämolysereagenz behandelt wird, das ein ionisches Detergens mit einem pH von 5.0 bis 9,5 enthält.

   b) in der hämolysierten Probe der Gesamthämoglobingehalt bestimmt wird und

   c) in derselben hämolysierten Probe das glykierte Hämoglobin immunologisch bestimmt wird.

6. Methode gemäß Anspruch 5, dadurch gekennzeichnet, daß der Gesamthämoglobingehalt durch Messung der charakteristischen Absorption des spezifischen Hämoglobin-Chromophor bestimmt wird.

7. Methode gemäß einem der Ansprüche 5 und 6, dadurch gekennzeichnet, daß das Hämolysereagenz zusätzlich ein nichtionisches Detergens enthält.

8. Methode gemäß einem der Ansprüche 5 - 7, dadurch gekennzeichnet, daß die hämolysierte Probe mit einem Reaktionspuffer versetzt wird, der ein nichtionisches oder zwitterionisches Detergens enthält und in der resultierenden Mischung das Gesamthämoglobin und das glykierte Hämoglobin bestimmt wird.

9. Testkit zum immunologischen Nachweis von glykiertem Hämoglobin bestehend aus mindestens zwei voneinander getrennten Verpackungen, von denen die eine ein Hämolysereagenz enthält, das ein ionisches Detergens enthält und dessen pH-Wert auf 5,0 bis 9,5 mit einem geeigneten Puffer eingestellt wurde, und die andere ein weiteres Reagenzgemisch, das die zum immunologischen Nachweis des glykierten Hämoglobins notwendigen Reagenzien sowie zusätzlich ein nichtionisches oder zwitterionisches Detergenz enthält.

10. Testkit gemäß Anspruch 9, dadurch gekennzeichnet, daß die zum immunologischen Nachweis notwendigen Reagenzien ganz oder teilweise auf einem Testträger aufgebracht sind.

**Claims**

1. Method for the determination of the content of glycated haemoglobin derivative in a blood sample, wherein

   (a) the blood sample is treated for up to 10 minutes with a haemolysis reagent with a pH of 5 to 9.5 which contains an ionic detergent at temperatures of 4 to 37°C and

   (b) the glycated haemoglobin is determined immunologically in the haemolyzed blood sample.

2. Method as claimed in claim 1, wherein the haemolysis reagent in addition contains a non-ionic detergent.

3. Method as claimed in one of the claims 1 - 2, wherein a reaction buffer which contains a non-ionic or zwitterionic detergent is added to the haemolyzed sample and the glycated haemoglobin is determined immunologically in the resulting mixture.

4. Method as claimed in one of the claims 1 - 3, wherein the glycated haemoglobin is HbA$_{1c}$.

5. Method for the determination of the content of a glycated haemoglobin and of the total haemoglobin from an identical blood sample, wherein

   (a) the blood sample is treated with a haemolysis reagent which contains an ionic detergent with a pH of 5.0 to 9.5,

   (b) the total haemoglobin content is determined in the haemolyzed sample and

   (c) the glycated haemoglobin is determined immunologically in the same haemolyzed sample.

6. Method as claimed in claim 5, wherein the total haemoglobin content is determined by measuring the characteristic absorbance of the specific haemoglobin chromophore.

7. Method as claimed in one of the claims 5 and 6, wherein the haemolysis reagent in addition contains a non-ionic detergent.

8. Method as claimed in one of the claims 5 - 7, wherein a reaction buffer which contains a non-ionic or zwitterionic detergent is added to the haemolyzed sample and the total haemoglobin and the glycated haemoglobin are determined in the resulting mixture.

9. Test kit for the immunological detection of glycated haemoglobin consisting of at least two separate packages one of which contains a haemolysis reagent which contains an ionic detergent and whose pH has been adjusted to 5.0 to 9.5 with a suitable buffer and the other contains a further reagent mixture which contains the reagents necessary for the immunological detection of the glycated haemoglobin and additionally a non-ionic or zwitterionic detergent.

10. Test kit as claimed in claim 9, wherein the reagents necessary for the immunological test are applied wholly or partially to a test carrier.

**Revendications**

1. Procédé pour déterminer la teneur d'un échantillon de sang en hémoglobine glucosée, caractérisé en ce que

   a) on traite l'échantillon de sang avec un réactif d'hémolyse possédant un pH de 5 à 9,5 à des températures de 4 à 37°C pendant un laps de temps s'étendant jusqu'à 10 minutes, le réactif contenant un détergent ionique, et

   b) on détermine par voie immunologique la teneur en hémoglobine glucosée dans l'échantillon de sang hémolysé.

2. Procédé selon la revendication 1, caractérisé en ce que le réactif d'hémolyse contient en outre un détergent non ionique.

3. Procédé selon l'une quelconque des revendications 1-2, caractérisé en ce qu'on mélange avec l'échantillon hémolysé, un tampon réactionnel qui contient un détergent non ionique ou zwitterionique et on détermine par voie immunologique la teneur en hémoglobine glucosée dans le mélange résultant.

4. Procédé selon l'une quelconque des revendications 1-3, caractérisé en ce que l'hémoglobine glucosée est $HbA_{1c}$.

5. Procédé pour la détermination de la teneur d'une hémoglobine glucosée et de l'hémoglobine totale à partir d'un échantillon de sang identique, caractérisé en ce que

   a) on traite l'échantillon de sang avec un réactif d'hémolyse qui contient un détergent ionique possédant un pH de 5,0 à 9,5,

   b) dans l'échantillon hémolysé, on détermine la teneur en hémoglobine totale, et

   c) dans le même échantillon hémolysé, on détermine par voie immunologique la teneur en hémoglobine glucosée.

6. Procédé selon la revendication 5, caractérisé en ce qu'on détermine la teneur en hémoglobine totale par mesure de l'absorption caractéristique du chromophore spécifique pour l'hémoglobine.

7. Procédé selon l'une quelconque des revendications 5 et 6, caractérisé en ce que le réactif d'hémolyse contient en outre un détergent non ionique.

8. Procédé selon l'une quelconque des revendications 5-7, caractérisé en ce qu'on mélange avec l'échantillon hémolysé un tampon réactionnel qui contient un détergent non ionique ou zwitterionique et on détermine dans le mélange résultant la teneur en hémoglobine totale et la teneur en hémoglobine glucosée.

9. Nécessaire d'essai pour la détection immunologique d'hémoglobine glucosée, constitué par au moins deux conditionnements séparés l'un de l'autre, l'un contenant un réactif d'hémolyse qui contient un détergent ionique et dont le pH a été réglé à une valeur de 5,0 à 9,5 avec un tampon approprié, l'autre contenant un mélange de réactifs supplémentaire qui contient les réactifs requis pour la détection immunologique de l'hémoglobine glucosée, et en outre un détergent non ionique ou zwitterionique.

10. Nécessaire d'essai selon la revendication 9, caractérisé en ce que les réactifs requis pour la détection immunologique sont appliqués en tout ou en partie sur un support d'essai.

FIG. 1

FIG. 2

FIG. 3

EP 0 559 164 B1

FIG. 4

FIG. 5

FIG. 6

FIG. 7

FIG. 8

HbA1c (g/dl)